# EUROPÄISCHE PATENTSCHRIFT

(11) **EP 1 635 776 B1**
(45) Veröffentlichungstag und Bekanntmachung des Hinweises auf die Patenterteilung: **10.09.2008**
(21) Anmeldenummer: 03817066.8
(22) Anmeldetag: 19.12.2003
(51) Int. Cl.: A61K 8/31, A61K 8/92, A61K 8/34, A61K 8/37, A61K 8/64, A61K 8/65, A61K 8/81, A61Q 19/00

(54) **LIPID-TRANSFERPROTEIN - SYSTEME UND DEREN KOSMETISCHE DERMATOLOGISCHE ANWENDUNG**
LIPIDIC PROTEIN-TRANSFER SYSTEMS AND COSMETIC/DERMATOLOGICAL USE THEREOF
SYSTEMES DE PROTEINES DE TRANSFERT LIPIDIQUE ET LEUR APPLICATION DERMATOLOGIQUE/COSMETIQUE

(30) Priorität: 28.05.2003 DE 10324256
(43) Veröffentlichungstag der Anmeldung: 22.03.2006
(73) Patentinhaber: Merz Pharma GmbH & Co. KGaA, 60318 Frankfurt Main (DE)
(72) Erfinder: BEUTLER, Rolf, D., 64739 Höchst/Hummetroth (DE); SCHMIDT, Karl-Heinz, 72810 Gomaringen (DE); NÜRNBERG, Eberhard, 91080 Uttenreuth/Weiher (DE); SCHRADER, Karl-Heinz, 37603 Holzminden (DE)
(74) Vertreter: Müller, Claudia
(86) Internationale Anmeldenummer: PCT/EP2003/014648
(87) Internationale Veröffentlichungsnummer: WO 2004/105716

(56) Entgegenhaltungen:
- EP-A- 0 504 043
- EP-A- 1 360 955
- WO-A-00/64472
- WO-A-99/36051
- CH-A- 686 554
- DE-A- 10 061 419
- DE-A- 19 813 057
- FR-A- 2 826 577

## Beschreibung

### Gegenstand der Erfindung

Die vorliegende Erfindung betrifft Zusammensetzungen mit bioaktiven Lipidtransferproteinen in stabilen Trägersystemen. Derartige Systeme sind vor allem geeignet zur Beeinflussung / Änderung von Lipidzellstrukturen durch verbesserten Lipidaustausch bei gleichzeitig hoher Stabilität. Dabei werden neben Transferproteinen und Lipidkomponenten insbesondere Schutzkolloide eingesetzt, durch welche eine Beeinträchtigung der Transferwirkung der Proteine und ein Verlust an Stabilität vermieden werden kann. Die Zusammensetzungen eignen sich vor allem zur extern - topischen Anwendung zur Reinigung, Pflege, dermatologischen und / oder therapeutischen Behandlung der Haut, Haar oder Schleimhaut.

### Hintergrund der Erfindung

Wirkstoffsysteme für den Lipidaustausch von und zu Zielstrukturen sind aus der DE- PS 3815 473 und den parallelen Schutzrechten FR 2631236, US -A-5776470, US-A 6077520 bekannt. Diese sind dadurch gekennzeichnet, dass mindestens eine amphiphile und / oder lipophile Komponente mit mindestens einem wasserlöslichen Transferprotein das Wirkstoffsystem bilden. Als Grundlagen zur Applikation auf die Haut werden allgemein Emulsionen bzw. Cremes erwähnt. Spezielle Formulierungen sind jedoch nicht offenbart.

Bekannt sind auch Lipoprotein- Cremes in Form von O/W-Emulsionen, die als Öl-Komponente wenigstens ein polares Öl und als Emulgator ein Protein aus Pflanzen (z. B. ab 3 bis 10 Gew.%) enthalten, vgl. EP- B- 1 047 396 Bevorzugte Emulgatoren sind Lipoproteine aus Hafer, Weizen oder Erbsen. Die (Ölkomponenten (1 bis 20%) müssen bestimmte Grenzflächenspannungen aufweisen und sind daher vorzugsweise ausgewählt aus C₂-C₆-Alkanolen oder der C₈-C₂₂-Alkanolestern einwertiger oder mehrwertiger C₂-C₆-Hydroxy-Alkanole oder der C₈-C₂₂-Alkanolester einwertiger oder mehrwertiger C₂-C₆-Hydroxycarbonsäuren. In derartigen Formulierungen sollen keine ionischen und hydrophilen O/W - Emulgatoren mit einem HLB- Wert von 5 und darüber eingesetzt werden. Als Zusatzstoffe können übliche galenische Hilfsstoffe wie Verdicker (Pflanzengumme, Celluloseether, synthetische und natürliche Polymere), sowie Co- Emulgatoren wie Cetyl- / Stearylalkohol (0,1 bis 1 Gewichtsteil pro Gewichtsteil Ölkomponente), Wirk- und Pflegestoffe enthalten sein.

Topisch applizierbare Produkte, enthaltend klassische hydrophile O/W-Emulgatoren sind aus der EP-A 0 532 465 bekannt. Dort werden ganz bestimmte Leguminosenfraktionen (Soja, Erbsen, nicht Getreide) eingesetzt zur Hautpflege, insbesondere zur Behandlung entzündlicher Hauterkrankungen.

Gemäß dem europäischen Patent EP-B 0 835 674 werden aus entsprechenden Vorrichtungen als schäumende O/W- Emulsionen erhältliche Cremes aus einer Fettphase, Wasser und 0,1 bis 2% eines gelbildenden Systems aus vernetzten Acrylat - Homopolymeren oder vernetzten Acrylat -Copolymeren hergestellt. Dabei sollen maximal 2 % übliche O/W- Emulgatoren wie Natriumlaurylsulfat enthalten sein. Als emulgierende Polymere eignen sich Acrylat/Acrylatcopolymere wie Pemulen®.

In einer Übersicht von R. Daniels, Pharm. Ztg. 147, (24.10.2002) werden solche Hydrodispersionsgele allgemein beschrieben. Im Gegensatz zu den "üblichen" Emulsionen erfolgt die notwendige Stabilisierung bei diesen Produkten wie erwähnt nicht mit sog. klassischen Emulgatoren, sondern mit Hilfe geeigneter Makromoleküle, d. h. mit Gelbildnern (siehe oben, EP-B 0835 674). Der Polymerzusatz bewirkt einen Verdickungseffekt und verleiht der Außenphase eine Fließgrenze. Derartige Systeme werden auch als Quasi-Emulsionen bezeichnet. Die genannten Hydrodispersionsgele enthalten als Polymer, z.B. Carbomer, und ein Silikonöl in Form von Phenyl Trimethicone oder auch Capryl/Caprinsäure - Triglyceride oder andere Öle. Kennzeichnend für die in der oben genannten Publikation beschriebenen Systeme ist die hier Abwesenheit von polareren Substanzen wie Partialester mehrwertiger Alkohole oder höherer Fettalkohole. Ebenso werden keine Proteinhaltigen Produkte als Inhaltsstoffe beschrieben.

Die DE 100 61 419 betrifft die Verwendung von Proteinhydrolysaten als Wirkstoff zur Restrukturierung keratinöser Fasern von Haaren.

In der CH B 686 554 werden kosmetische Mittel beschrieben aus Proteinhydrolysaten ohne weitere Charakterisierung und Wasser / Glycerin. Derartige Mittel werden auf herkömmliche Weise in kosmetische Produkte eingearbeitet.

In der US- Patentanmeldung US A1 2002/0054918 werden pharmazeutische Wasserstoffperoxid haltige Mittel zur Behandlung entzündlicher Hautpartien beschrieben. Dabei können ggf. Feuchtigkeitsmittel wie Hyaluronsäure, Weizenprotein ohne weitere Charakterisierung, Glycerin oder Dimethicon, Tocopherol zusätzlich verwendet werden z. B. bei der Herstellung von Lotionen, Cremes, Emulsionen oder Salben auf herkömmliche Weise.

Die WO 00/64472 beschreibt Zusammensetzungen zur Behandlung dermatologischer Störungen, enthaltend Fruchtextrakte wie Granatapfelextrakt, um freie Radikale zu neutralisieren. Als Träger für den Fruchtextrakt werden bekannte Grundlagen wie Stärke, Polyvinylpyrrolidon u. ä. beschrieben. Daneben können Feuchtigkeitsmittel wie Hydroxypolycarboxylsäuren, Dimethicone, Glycerin , Natriumchlorid oder Weizenprotein ohne spezielle Charakterisierung eingesetzt werden.

Insgesamt ergibt sich hieraus, dass Proteinhydrolysate oder Proteine bisher als Wirkstoffe, insbesondere Feuchthalter, in kosmetischen Produkten zur Verbesserung von Haut oder Haar eingesetzt wurden. Dabei wurden herkömmliche kosmetische Formulierungen, insbesondere unter Einsatz herkömmlicher, vor allem O/W- Emulgatoren, hergestellt.

### Aufgabe der Erfindung

Angesichts der unerwünschten Eigenschaften von Emulgatoren vor allem des klassischen O/W- Typs, wie ionische Substanzen wie z. B. Natriumlaurylsulfat u.ä. oder auch nichtionische O/W-Emulgatoren vom Typ der Macrogolabkömmlinge mit höheren Alkoholen oder -fettsäuren sollen Zusammensetzungen, - für insbesondere extern, d.h. topisch applizierbare Produkte - bereitgestellt werden, die trotz des Fehlens derartiger klassischer Emulgatoren stabil sind und wobei Proteine als Lipidtransfer - Komponenten eingesetzt werden können, ohne dass deren Wirksamkeit z. B. infolge einer emulgierenden Wirkung beeinträchtigt wird.

### Erläuterung der Erfindung

Diese Aufgabe wird erfindungsgemäß gelöst durch eine Zusammensetzung, welche wenigstens ein Lipidtransferprotein sowie wenigstens eine lipophile und / oder amphiphile Komponente sowie Wasser enthält und darüber hinaus wenigstens ein emulgierendes Schutzkolloid aufweist, das ausgewählt ist aus natürlichen Hydrokolloiden, insbesondere der Gruppe umfassend Caseinate, tierische Proteinderivate, Gelatine, Albumine, tierisch - marine Kolloide und

Derivaten hiervon, oder der Gruppe aus synthetisch / partial synthetisch hergestellten Kolloiden oder Mischungen hiervon.

Hierdurch wird einerseits ein stabiles Emulsionssystem gebildet, worin die Transferproteine als solche ihre gewünschte katalytische Wirkung entfalten, ohne in das emulgierende System einzugreifen. Andererseits werden Schutzkolloide eingesetzt, welche sowohl eine stabile Emulsion, z. B. als Creme gewährleisten als auch eine Emulgierwirkung der Transferproteine überflüssig machen. Diese wäre dem beabsichtigten Zweck kontraindiziert, da ansonsten die Strukturen emulgiert würden, die transferiert werden sollen, nämlich die Lipidkomponenten. Mit Hilfe der besonderen Schutzkolloide ist es daher überraschenderweise gelungen, einen verbesserten Lipidtransfer zu ermöglichen, wodurch der Lipidhaushalt der Zellen, insbesondere der Haut, zum einen ausgeglichen und zum anderen besser mit lipidischen Wirkstoffen versorgt werden kann. Dabei kann auf klassische O/W- (=hydrophile) Emulgatoren verzichtet werden.

Somit ist die erfindungsgemäße Kombination, d. h. das System aus lipophilen und / oder amphiphilen Komponenten, Lipidtransferproteinen und Schutzkolloiden für breite Anwendungsgebiete geeignet , insbesondere als extern / topisch applizierbares Kosmetikum oder Dermatikum, auch unter Einsatz von Zusatzstoffen. Die Zusammensetzung kann insofern als Creme, Lotion, Milch z. B. in Form einer W/O-, O/W-, insbesondere vor allem einer O/W -Zusammensetzung, Salbe, Gel, Spray u. ä. vorliegen, je nach Anteil der einzelnen Komponenten bzw. Wassergehalt. Insbesondere kann die Zusammensetzung verwendet werden zur Herstellung von Mitteln zur Beeinflussung von Lipidzellstrukturen, insbesondere zur Reinigung, Pflege, Sonnenschutz und/ oder der prophylaktischen oder therapeutischen Behandlung der Haut von Säugern, vor allem zur Verbesserung der Hautzellstruktur von alternder, geschädigter, insbesondere Licht geschädigter, trockener, empfindlicher, entzündlicher Haut, zum Schutz gegen Sonne von Säugern bzw. auch als Duschpräparat, Pflegecreme oder Lotion.

Die erfindungsgemäße Zusammensetzungen können auch auf die Schleimhaut appliziert werden, wobei ebenfalls ein besonders guter Lipidtransfer möglich ist.

Die Mengenangaben beziehen sich auf Gew.%, soweit nichts anderes angegeben.

Besonders geeignete Zusammensetzungen umfassen Proteine aus tierischen pflanzlichen, marinen mikrobiologischen Quellen wie Getreide, Knollenfrüchten, Milch, Seide, insbesondere tierischen Quellen, wie nachfolgend beschrieben.

Die Lipide sind vor allem ausgewählt aus Lecithinen, Cholesterinen, Phospholipiden, Sphingolipiden, Gangliosiden, Cerebrosiden, Ceramiden lipophilen oder amphiphilen Pflanzenstoffen, Glyceriden, Kohlenwasserstoffen, natürlichen oder synthetischen Fetten, Ölen, Wachsen, Fettalkoholen, Fettsäureestern, Fettsäurepartialestern, Silikonölen, Silikonwachsen oder Mischungen hiervon, insbesondere aus Kombinationen aus wenigstens einer Substanz aus der Gruppe der stärker polaren Lipide /Amphiphile in Kombination mit wenigstens einer Substanz aus der Gruppe der schwächer polaren Lipide / Amphiphilen, wie nachfolgend beschrieben.

Bevorzugt weist die Zusammensetzung Wasser, z. B. 0,1 bis 85 %, insbesondere 10 bis 60 % auf. In einer anderen Ausführungsform können vor allem auch 35 bis 70% Wasser vorhanden sein.

### Nähere Beschreibung der Inhaltstoffe

### 1) Lipidtransferproteine

Die erfindungsgemäßen Lipidtransferproteine werden bevorzugt ausgewählt aus hydrolpsiertem Milchprotein, in Kombination mit Haferprotein bzw. Hydrolysat.

Zu den geeigneten Proteinen gehören insbesondere solche Lipidtransferproteine aus Getreide, vor allem die aus Hafer erhältlich sind, mit einer mittleren Molekülmasse von 4000 bis 10 000 D, Proteine aus Milch mit einer mittleren Molekülmasse von 2000 bis 8000 D, oder Mischungen hiervon.

Ferner eignen sich vor allem vorteilhafter Weise auch tierische Proteine aus Milch sowie entsprechende Hydrolysate (mittlere Molekülmasse, vor allem 2000 bis 4000 D).

D Hierunter sind ganz besonders Getreideproteine und insbesondere Milchproteine geeignet.

Besonders geeignet sind auch Mischungen von Lipidtransferproteinen wie z. B. Mischungen aus pflanzlichen Proteinen und tierischen Proteinen.,̅

Die Proteine können auf bekannte Weise durch Aufschlämmung in Wasser, Zentrifugation und Chromatographie erhalten werden, wobei weitere dem Fachmann bekannte Aufarbeitungsmethoden wie Homogenisierung, Entfettung mit organischen Lösungsmitteln, Ausfällen, Dialyse, Filtration zur Anwendung kommen können, vgl. die vorgenannte US-A- 5,776,470. Weitere Methoden zum Erhalt von Proteinen dieser Art sind z. B. in der EP- A 0 620 979 und darin genannter Literatur beschrieben.

Auf diese Weise werden Proteine bzw. Hydrolysate hiervon mit den genannten Molekülmassen gewonnen, die dann in den erfindungsgemäßen Zusammensetzungen eingesetzt werden. Sie weisen eine insbesondere katalytische Transferaktivität auf, welche nach dem bekannten Resonanz - Energie - Transfer Test (RET- Test) bestimmt werden kann, vgl. Nichols, J.W., Pagano R.E., J. Biol. Chem. 258 (1983), 5368-5371. Demnach können beispielsweise zwei Liposomenspezies unterschiedlicher Lipidzusammensetzung eingesetzt werden, wobei eine als Sonde verwendet wird und das Resonanz - Energie- Transfer- System zwei in Resonanz stehenden Fluorophoren, wie z. B. Nitrobenzoxadiazol und Rhodamin B enthält. Bei räumlicher Nähe der beiden Fluorophore in den Sondenliposomen verschwindet die Eigenfluoreszenz des angeregten Fluorophors, da die Energie vom zweiten Fluorophor unmittelbar absorbiert wird. Wird nun durch Protein - katalytischen Lipidtransfer der Abstand zwischen den beiden Fluorophoren verändert, so tritt die Eigenfluoreszenz des angeregten Fluorophors wieder auf. Sie ist ein direktes Maß für den Lipidtransfer.

Insbesondere sind die erfindungsgemäß eingesetzten Proteine zumindest wasserdispergierbar, vor allem aber wasserlöslich. Sie wirken vor allem als Transfervehikel. Sie können in Mengen von 0,01 bis 10 Gew.%, vor allem 0,01 bis 8 Gew. %, eingesetzt werden.

Bevorzugt werden Mengen von 0,01 bis 5 Gew. %, insbesondere 0,01 bis 2 Gew. % , vor allem 0,01 bis 0,95 Gew. %, und ganz besonders 0,01 bis 0,85 oder auch 0,1 bis 0,85, insbesondere 0,1 bis 0,5 Gew. % gewählt.

Werden Kombinationen eingesetzt, insbesondere aus tierischen Proteinen und pflanzlichen Proteinen der genannten Art , kann dies z. B. im Verhältnis 10:1 bis 1:10, insbesondere 5:1 bis 1:1 erfolgen. Vor allem geeignet ist Milchprotein, insbesondere hydrolysiertes Milchprotein in Kombination mit Pflanzenprotein (z. B. Haferprotein bzw. Hydrolysat hiervon.

### 2. Lipidkomponenten

Als Lipidkomponenten, nämliche lipophile oder amphiphile Komponenten, sind sowohl stärker polare Substanzen als auch schwächer polare Substanzen geeignet.

So können vor allem aus der Gruppe der schwächer polaren Substanzen natürliche oder synthetische (gesättigte, ungesättigte) Fette /Öle bzw. Wachse eingesetzt werden. Hierzu gehören natürliche, partialsynthetische oder synthetische Öle, Kohlenwasserstoffe wie Squalen, Squalan, insbesondere auch flüssige Paraffine, Isoparaffine, Dioctylcyclohexane, Isodecan, Isohexadecane.

Auch Fettsäureester, z.B. C₂₋₁₈-Alkoholfettsäureester wie Isopropylfettsäureester (Palmitat, Myristat, Isostearat, Oleat), Decyl Oleate, Hexyl Laurate, C 12 - 15 Alkyl Benzoate , Dicaprylyl Carbonate sowie verzweigte Fettsäureester wie Cetearyl Octanoate, Di-n-Butyladipate sind geeignet;

Weiterhin sind C₈₋₂₂- Fettalkoholether wie Dicaprylyl Ether oder Fettalkoholester wie C₁₂₋₁₃- Alkyl Lactate, insbesondere Glyceride (C₁₂₋₂₂ Fettsäure-Glyceride) wie Triglyceride, insbesondere mittelkettige (Neutralöle) wie Caprylic/Capric Triglyceride sowie deren Polyolester wie Propylene Glycol Dicaprylate/ Dicaprate geeignet;

Auch Natürliche (gesättigte/ungesättigte) Fette und Öle finden Anwendung wie Sonnenblumen-, Soja-, Pfirsichkem-, Aprikosenkern-, Traubenkem-, Ricinus-, Erdnuß-, Mandel-, Nerz-, Weizenkeim-, Sesam- Distel- , Avocadoöl, Shea Butter oder Illipé Butter; Natürliche flüssige Wachse, z.B. Jojobaöl oder dessen Substitut Oleyl Erucate;

Weiterhin geeignet sind auch Silikonderivate wie Silikonöle und -wachse, z.B. Polydimethylsiloxane wie Dimethicone, Cyclomethylsiloxane wie Cyclopentasiloxane, Phenylmethylpolysiloxane wie Phenyl Dimethicone oder Alkyl-Polymethylsiloxan-Copolymere wie Cetyl Dimethicone, Stearyl Dimethicone Dialkoxydimethylpolysiloxane wie Stearoxy Dimethicone, Behenoxy Dimethicone;

Geeignet sind auch Wachse wie natürliches oder gebleichtes Bienenwachs (C₂₀₋₄₀- Alkylstearat) oder Micro Wax oder Kohlenwasserstoffwachse wie Mineral Wax sowie hydriertes Rizinusöl oder synthetische Wachse wie Cetylpalmitat oder Myristylmyristat, oder Stearylstearate .

Als stärker polare Lipid - Komponenten sind C₈₋₂₂-Fettalkohole wie - Oleylalkohol, Octyldodecanol, Cetyl/stearylalkohol; geeignet;
Auch C₁₂₋₂₂ - Fettsäurepartialester von mehrwertigen C₂₋₆- Alkoholen wie insbesondere Monoglyceride, oder Diglyceride z. B. Glycerolmono / Distearat, Mischungen hiervon; Polyol C₁₂₋₂₂ - Fettsäureester wie PEG-7- Glyceryl Cocoate, propylen Glykol Dicaprylate /dicaprate,Gemische wie Hexyldecanol und Hexyldecyl Laurat können eingesetzt werden.

Besonders geeignet sind hier auch liposomale Lipidkomponenten oder Derivate hiervon wie insbesondere Lecithin wie Soja- Eilecithin; Phospholipide, wie Phosphatidylcholin, Phosphatidylserin, Phosphatidylethanolamin, Phophatidylglycerol, Phosphatidylinositol, Ganglioside, Cerebroside, Cholesterin und Ceramide, wie z.B. Ceramid-2, -3, -6 sowie Sphingolipide.

Ganz besonders bevorzugt werden die Lipidkomponente(n) ausgewählt aus den genannten Kohlenwasserstoffen, (C₈₋₂₂-) Fettalkoholen, -ethern, (C₂₋₆Alkohol)- (C₁₂₋₂₂) Fettsäurepartialester, (Polyol- C₁₂₋₂₂-) / C₂₋₁₈- Fettsäureester, Mono- Di/ Tri (C₁₂₋₂₂-) Glyceriden, natürlichen oder synthetischen Ölen oder Fetten, Lecithinen, Phospolipiden, Ceramiden, Sphingolipiden, Gangliosiden, Cerebrosiden, Cholesterinen, Silikonölen, Silikonwachsen, lipophile oder amphiphile Pflanzenstoffen oder Mischungen hiervon.

Insbesondere sind flüssige Paraffine, natürliche Fette/Öle/Wachse, Mono-/Triglyceride von C₁₂₋₂₂- Fettsäuren, C₁₂₋₂₂Fettalkohole, C₁₂₋₂₂-Fettsäurepartialester von C₂₋₆-mehrwertigen Alkoholen , Polyol C₁₂₋₂₂-Fettsäureester Lecithine, Phospholipide, hierunter vor allem die oben erwähnten, Ganglioside, Cerebroside, Cholesterin, Ceramide, Sphingolipide, Polysiloxanverbindungen, lipophile oder amphiphile Vitamine und Mischungen hiervon geeignet.

Es hat sich ferner gezeigt, dass eine besonders vorteilhafte Ausgestaltung erfindungsgemäßer Zusammensetzungen dann erhalten wird, wenn die lipophilen / amphiphilen Substanzen eine Kombination aus einer oder mehreren stärker polaren Substanzen und einer oder mehreren schwächer polaren Substanzen darstellen. Zu den schwächer polaren, lipophilen Substanzen zählen Kohlenwasserstoffe, natürliche oder synthetische Fette oder Öle oder Wachse, Triglyceride, Silikonderivate wie beschrieben, insbesondere Polysiloxanverbindungen. Zu den stärker polaren Substanzen gehören Lecithine, Ceramide, Cholesterine, Phospholipide, Sphingolipide, Ganglioside, Cerebroside, lipophile oder amphiphile Pflanzenstoffe, Mono- /Diglyceride von C₁₂₋₂₂- Fettsäuren, C₁₂₋₂₂Fettalkohole, C₁₂₋₂₂-Fettsäurepartialester von mehrwertigen C₂₋₆- Alkoholen, C₂₋₆- Polyol- C₁₂₋₂₂- Fettsäureester. Erifurging s.10

Werden je einer oder mehrere Vertreter der stärker polaren Gruppe und je ein oder mehrere Vertreter der schwächer polaren Gruppe kombiniert, so führt dies in unerwarteter Weise zusammen mit dem oder den Lipidtransferprotein/en zu einem möglicherweise aufgrund einer Adduktbildung noch besseren Lipidtransfer.

In einer weiteren bevorzugten Ausführungsform werden diese Lipide bzw. Lipidkombinationen mit den Transferproteinen mit einer mittleren molaren Masse von 2000 bis 30000 D zusammen eingesetzt, insbesondere solchen, ausgewählt aus tierischen oder tierischen und pflanzlichen Transferproteinen.

Ganz besonders bevorzugt sind Kombinationen aus liposomalen Lipidkomponenten, Mono- /Triglyceriden von C₁₂₋₂₂-Fettsäuren, C₁₂₋ In bevorzugten Zusammensetzungen ist / sind die lipophile und / oder amphiphile Komponente(n) ausgewählt aus Lecithinen, Sphinglipiden, Cholesterinen, Phospholipiden, Sphingolipiden, Gangliosiden, Cerebrosiden, Ceramiden lipophilen oder amphiphilen Pflanzenstoffen, Glyceriden, Kohlenwasserstoffen, natürlichen oder synthetischen Fetten, Ölen, Wachsen, Fettalkoholen, Fettsäureestern, Fettsäurepartialestern, Silikonölen, Silikonwachsen oder Mischungen hiervon.

Es ist weiterhin bevorzugt, wenn die lipophile und / oder amphiphile Komponente(n) ausgewählt ist (sind) aus je einer oder mehreren Substanzen der Gruppe aus Kohlenwasserstoffen, natürlichen oder synthetischen Fetten, Ölen, Wachsen, Fettsäureester, Fettalkoholether, Polysiloxanverbindungen, Triglyceride, in Kombination mit je einer oder mehreren Substanzen aus der Gruppe aus Lecithinen, Sphingomyelinen, Cholesterinen, Phospholipiden, Sphingolipiden, Gangliosiden, Cerebrosiden, lipophilen oder amphiphilen Pflanzenstoffen, Mono- /Diglyceride von C₁₂₋₂₂- Fettsäuren, C₁₂₋₂₂Fettalkohole, C₁₂₋₂₂-Fettsäurepartialester von C₂₋₆-Alkoholen, Polyol - C₁₂₋₂₂- Fettsäureester. ₂₂Fettalkoholen und Fettsäureestern /Partialestern wie beschrieben, Vitaminen und Kohlenwasserstoffen.

Ganz besonders bevorzugt können auch Silikonderivate wie beschrieben, insbesondere Polysiloxane, vor allem in Verbindung mit o.g. Kombinationen eingesetzt werden.

Die lipophile(n) / amphiphile(n) Komponente(n) liegen in Mengen von 0,1 bis 40 Gew.%, bevorzugt 1 bis 30, und ganz besonders 1 bis 20 Gew.% vor.

### 3. Schutzkolloide

Das oder die erfindungsgemäß eingesetzten Schutzkolloide sind solche natürlichen Ursprungs, nämlich tierischen oder tierisch - marinen Ursprungs und Derivaten hiervon oder synthetische oder (partial)synthetisch hergestellte Kolloide, insbesondere Hydrokolloide. Diese sind insbesondere neutral nicht ionisch oder ionisch. Besonders bevorzugt sind solche natürlichen Ursprungs, vor allem tierischen Ursprungs. Ferner sind auch Kombinationen von Vertretern dieser Gruppen (z. B. tierisch /synthetisch und / oder partialsynthetisch oder mehrere aus einer Gruppe), sehr gut geeignet, um ebenso wie einzeln quasi in der Zusammensetzung als Emulgator zu wirken.

Insbesondere geeignet sind insofern besonders aus der Gruppe der natürlichen Vertreter (tierische) Caseinate, Proteinderivate tierischer Natur, Gelatine, Albumine, oder Mischungen hiervon.

Als Proteinderivate tierischer Natur (oder entsprechend synthetisch hergestellte Produkte) eignen sich Hydrolysate mit einer höheren mittleren Molmasse .

Ganz besonders bevorzugt sind als Schutzkolloide (natürlicher, tierischer Herkunft) wasserlösliche Caseinverbindungen, insbesondere Salze von Casein, wie Natriumcaseinat. Die relativen Molmassen können insbesondere im Bereich von 15000 bis 30000 D liegen.

Als Gelatine eignen sich die Typen A und B gemäß Ph.Eur. 4. Ebenfalls geeignet sind Albumine aus Ei, Serum, die chromatographisch aus dem natürlichen Ausgangsmaterial gewonnen werden können.

Die genannten Verbindungen sind an sich bekannt.

Zu den synthetisch / partial synthetischen Kolloiden gehören bevorzugt wasserlösliche (Poly)(Meth)Acrylatpolymere wie Carbomere, vgl. Ph.Eur.4 oder wasserlösliche Cellulosederivate wie Methylcellulose, Hydroxypropylcellulose, Hydroxyethylcellulose, Hydroxypropylmethylcellulose.

Diese letztgenannten Kolloide weisen insbesondere eine mittlere molare Masse von mehr als 40 000 D , z. B. Carbomere 1,25 bis 4 Mio. auf.

Das oder die Schutzkolloide werden bevorzugt in Mengen von 0,01 bis 10 Gew.%, vor allem 0,01 bis 5 Gew.% eingesetzt, und vor allem 0,1 bis 4 Gew.%. In einer weiteren Ausführungsform können sie auch in Mengen von 0,1 bis 3 Gew. % vorliegen.

Die efindungsgemäßen Zusammensetzungen weisen bevorzugt neben diesen genannten drei Komponenten Lipid/ Transferprotein / Schutzkolloid noch Wasser auf, wobei dessen Menge von der galenischen Formulierung abhängt und insofern zwischen 0 und 80 %, insbesondere 5 und 75 %, weiterhin auch 35 bis 65 Gew.%, liegen kann.

Hierin können bevorzugt noch Zusatzstoffe, z. B. 0,01 bis 40 Gew. % vorhanden sein.

Besonders bevorzugt werden die Zusammensetzungen daher in Form von O /W- artigen Emulsionen wie Cremes hergestellt, wobei aber jede andere Formulierungsart möglich ist wie Gel, Salbe, Spray oder auch W/ O Emulsion.

Insbesondere bevorzugt sind Zusammensetzungen, welche 0,01 bis 10 Gew.% , insbesondere 0,01 bis 3 Gew.%, vor allem aber 0,1 bis 0,9 Gew.%, eines oder mehrerer Lipidtransferproteine, 0,01 bis 40 Gew.%, bevorzugt 0,1 bis 25 Gew.% einer oder mehrerer lipophiler und / oder amphiphiler Komponenten, insbesondere 1 bis 20 Gew.%, ferner 0,01 bis 10 Gew.%, insbesondere 0,1 bis 6 Gew. % eines oder mehrerer Schutzkolloide, sowie 0,01 bis 30 , bevorzugt 0,1 bis 20 Gew. % Zusatzstoffe und als Rest Wasser enthalten. Ganz besonders bevorzugt sind hier die erwähnten Lipidkombinationen aus stärker polaren und schwächer polaren Substanzen i. V. m. den Transferproteinen mit einer mittleren molaren Masse von 2000 bis 30 000 D.

### 4. Zusatzstoffe

Die erfindungsgemäßen Zusammensetzungen können wie erläutert, Zusatzstoffe in einer Menge von 0 bis 40%, insbesondere 0,01 bis 40 Gew.%, bevorzugt 0,1 bis 30 Gew. %, vor allem 0,1 bis 20 Gew. % aufweisen.

Zu den bevorzugten Zusatzstoffen gehören Wirkstoffe, Konditioniermittel Konservierungsmittel, Farbstoffe, Parfümstoffe, Pflegestoffe. Diese können insbesondere in Mengen von jeweils 0,01 bis 10%, bevorzugt ; .0,01 bis 5%, vorliegen.

Die Wirkstoffe können Wasser- oder fettlöslich sein und sind insbesondere ausgewählt aus Extrakten aus Pflanzen, diesen Extrakten entsprechenden synthetisch hergestellten Substanzen und analogen Derivaten hiervon, Vitaminen, sowie Haut beeinflussenden Wirkstoffen und Mischungen hiervon.

Extrakte aus Pflanzen sind beispielsweise solche aus Ylang Ylang, Kiefemnadel, Zypresse, Thymian, Minze, Limetten, Orangen, Grapefruit, Mandarine, Wacholder, Baldrian, Zitronenmelisse, Eukalyptus, Palmarosa, Rosmarin, Lavendel, Rosenholz, Lemongras, Fichtennadel, , Ingwer-, Johannisbeer-, Lindenblüten-, Magnolien-, Algen-, Aloe Vera-, Ananas-, Guave-, Echinacea-, Efeublätterxtrakt, Birkenblätterextrakt, , Ringelblumen- Hibiskus-, Klettenwurzel - Hamamelis-, Wassemabelkraut-, , Quitten-, Wasserlilien-, Zimtextrakt oder Mischungen hiervon.

Als Vitamine eignen sich z. B. Vitamin A, B, E, C bzw. geeignete Derivate hiervon wie Ester z.B. Palmitat, Acetat oder Phosphat.

Weiter geeignete Wirkstoffe sind Mineralstoffe und Spurenelemente wie Kupfer, Zink, Magnesium bzw. deren Derivate, vor allem Salze, wie Zincidone^{®} (Zink PCA), Zinkglukonat oder Kupfergluconat.

Weiterhin können adstringierende und sebumregulierende Substanzen eingesetzt werden wie Hydroxydecanoic Acid, Pyridoxine, Niacinamide, Glycerin, Caproyl Collagen Aminoacids, Sebacic Acid, Cinnamonum zeylanicum.

Es können auch kühlende/beruhigende Wirkstoffe wie Menthyl Lactate oder Sodium Hyaluronate, Wheat Germ Extrakt, Saccharomyces Cerevisiae Extrakt inkorporiert werden.

Darüber hinaus sind als Wirkstoffe durchblutungsfördernde Stoffe, z. B. Nikotinsäurederivate wie Methyl- oder Tocopherylnikotinat, Alpha- und

Betahydroxysäuren und deren Derivate, z. B. Glykol-, Äpfel-, Zitronen-, Wein-, Milchsäure, Salicylsäure, Isopropylbenzylsalicylate, C12 - 13 Alkyl Lactate Minoxidil oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. D-Panthenol, Panthenyltriacetat; Allatoin; Bisabolol; Azulene, z. B. Cham-Azulene oder Guaj-Azulen; Phytosphingosine; Triclosan; Chlorhexidin-Derivate einsetzbar.

Ferner können auch antioxidativ sowie zellschützend wirkende Stoffe wie Polyphenole, Flavonoide, z. B. Rutin, Ferulasäure und deren Ester oder Isoflavone wie Soja-Isoflavone oder Coenzym Q 10 , Hyaluronsäure, Oleanolsäure und /oder Ursolsäure als wirksame Zusatzstoffe eingesetzt werden.

Dagegen werden stark oxidative Substanzen wie insbesondere Wasserstoffperoxid nicht eingesetzt.

Weitere Wirkstoffe sind UV-Filter wie UVB, UVA und Breitbandfilter wie sie allgemein bekannt sind, z.B. Zimtsäureester, Salicylate, analoge bekannte Substanzen oder auch anorganische UV-Filter wie Zinkoxid und Titandioxid insbesondere mikronisiert und/oder beschichtet.

Weitere gebräuchliche Wirkstoffe sind Biotin, Allantoin, Panthenol, Niacinamid, Harnstoff, Inositol.

Je nach Anwendungszweck können auch öllösliche bzw. wasserlösliche Wirkstoffe für kosmetische und / oder dermatologisch/pharmazeutische Zwecke eingesetzt oder kombiniert werden.

Dermatologisch/ pharmazeutische Wirkstoffe können insbesondere antimikrobiell wirksame Substanzen (Antibiotika; Antimykotika, antivirale Stoffe), Hormone oder hormonwirksamen Stoffen wie Sexualhormonen, Wundreinigungsmitteln, Analgetika, Cytokine, Zytostatika, Antiaknemittel, Immunsuppressiva, Pigmentierungsregulatoren, Mittel gegen Photodermatosen oder oben genannte vasoaktive / antiphlogistische Substanzen oder Kombinationen hiervon sein.

Zu weiteren Zusatzstoffen gehören wie erwähnt übliche Konditioniermittel in geeigneten Mengen wie Acrylamide, Stärke, wie beispielsweise Methylcellulose, Hydroxyethylcellulose, Carmellose-Na, Hydroxypropylcellulose, Hypromellose oder Stärkeglycola, Reis-, Weizen-, Mais- und Kartoffelstärke, ferner auch hydrophobisch modifizierte Stärken (Corn Starch Modified), Aluminium Starch Octenylsuccinate, Hydroxypropyl Starch Phosphate Ester oder dessen Gemische wie Aluminium Starch Octenylsuccinate, Acrylates Copolymer, Magnesium Carbonate, und ähnliches geeignet.

Ferner sind hier auch Acrylate sowie Polysaccharide (z. B. Xanthan Gum), Cellulose-Derivate wie Hydroxypropyl Methyl Cellulose oder Ethylcellulose oder Silikate wie Magnesium Aluminium Silikat zu nennen.

Bekannte Acrylamide sind Polyacrylamid, oder Polyacrylamidhaltige Gemische. Als Acrylate können Carboxy-Vinyl-Mischpolymerisate mit hohem Molekulargewicht (1 - 3 Mio.) sowie deren Copolymere eingesetzt, insbesondere nach Neutralisierung durch Alkali, wie Carbomer, oder die bereits neutralisierten Carbomere wie Sodium Carbomer. Es können auch AcrylatCopolymere eingesetzt werden, z.B. Acylates/C10-30 Alkyl Acrylate Crosspolymer, oder Acrylamidalkylsulfonsäurederivate wie Ammonium AcryloyldimethyltaurateNP Copolymer.

Als Konditioniermittel können weiterhin auch Komplexbildner wie EDTA, Na-Salz, Mittel zur pH- Einstellung, z.B. Zitronensäure, Natronlauge Lösungsmittel wie Propylenglykol oder Alkohole, Salze wie NaCl, Stärke, bzw. Stärke-Derivate oder Mischungen hiervon enthalten sein.

Weitere Konditioniermittel sind insbesondere Mono- und Dialkylphosphate oder Glycerylstearat.

Besonders geeignete Pflegestoffe sind Phytosterole (im wesentlichen Gemische aus β-Sitosterol, Campesterol und Stigmasterol oder ethoxylierte Derivate hiervon) wie solche aus Rapsöl.Auch Lecithin (z.B. Sojalecithin). Phosphatidylcholin, Phosphatidylserin oder- diethanolamin, oder Mischunggen hiervon mit den oben genannten Phytosterolen sind hier verwendbar.

Weiterhin können hier Silikonöle z.B. Dimethicone oder Silikon -(Co)Polymere, z.B. Di- Cyclomethicon oder Divinyldimethicone/Dimethicone Copolymer, C12-13 Pareth-3/C12-13 Pareth-23 Verwendung finden.

Weitere Pflegestoffe sind Feuchthaltemittel wie Glycerin, Propylenglykol oder Polyethylenglykole, Butylenglykol, Sorbitol oder Polymere, z.B. Polyquaternium-Typen wie Polyquaternium -39 , Collagen oder dessen Hydrolysate, Aminosäuren, Harnstoff, Polysaccharide (Biosaccharide Gum-1), Glucosaminoglykane, z.B. Hyaluronsäure oder sulfatierte Glucosaminoglykane wie Chondroitinsulfat, Dermatansulfat, Keratansulfat, Heparansulfat, insbesondere deren Na-Salze oder Heparin-Na , Glukane, z.B. β-Glukan, z.B.

Hafer β-Glukan Mannane wie Konjac Mannane, oder handelsübliche Feuchthaltemittel wie Inositol, Salze, z.B. Natriumlactat, DL-2-Pyrrolidon-5-Carbonsäure, Na-Salz.

Bevorzugt sind Polyethylen-/ Propylenglykol oder Polysaccharid-Verbindungen, Algenextrakte und/oder Hyaluronsäure, Na-Salz.

Weitere mögliche Zusatzstoffe sind geeignete Tenside wie Sarcosinate, Acylglutamate, Isethionate, Polyglycoside.

Daneben können gegebenenfalls ein oder mehrere Konservierungsmittel vorhanden sein. Geeignete Konservierungsmittel sind beispielsweise lodopropynylbutylcarbamat, DMDM Hydantoin, Phenoxyethanol und weitere gebräuchliche Konservierungsstoffe, wie z.B. Sorbin- und Dehydracetsäure und deren Salze, Methyldibromoglutarononitril, etc. oder Kombinationen hiervon, oder andere Säuren wie Benzoe- oder Salicylsäure, oder Benzylalkohol oder Ester wie p-Hydroxy-benzoesäureester, z.B. Methyl-, Ethyl-, Propyl-, Butyl-, Iso-Butylparaben, bevorzugt Methyl-oder Propylparaben oder Mischungen hiervon oder Climbazol oder geeignete Kombinationen der genannten Stoffe wie z.B. Methyl-, Propylparaben und Sorbinsäure in Frage. Insbesondere geeignet sind Mischungen, z.B. aus DMDM Hydantoin und lodopropynylbutylcarbamat oder aus Phenoxyethanol mit p-Hydroxybenzoesäureester, Isothiazolinone, Methyldibromoglutanonitril, etc.

Geeignete Parfümstoffe (z.B. bevorzugt 0,01 bis 5%) sind z.B. die unter Wirkstoffen genannten etherischen Öle oder handelsübliche Parfümmischungen.

Als Farbstoffe (z.B. bevorzugt 0,01 bis 2%) werden vorzugsweise die für gattungsgemäße Produkte bekannten Komponenten gewählt.

Die Art und Menge der Zusatzstoffe richtet sich nach der gewünschten Anwendungsform und dem gewünschten Zweck. Es ist bevorzugt, wenn höchstens 30%, bevorzugt 0,01 bis 25%, vor allem auch 0,1 bis 25% oder 1 bis 20 %, insbesondere 3 bis 20% an Zusatzstoffen vorhanden sind.

Besonders bevorzugt sind 0,01 bis 25% Zusatzstoffe, welche vor allem ausgewählt sind aus Wirkstoffen, Pflegestoffen, Konditioniermitteln, Parfümstoffen, geeignet

Bevorzugt werden vor allem auch die genannten Vitamine, insbesondere Tocopherol oder Derivate hiervon und/oder Panthenol eingesetzt, insbesondere auch in Kombination mit Ursolsäure und /oder Oleanolsäure und / oder Hyaluronsäure (z. B. Natriumsalz), und / oder Q 10.

### Anwendung

Je nach beabsichtigtem Zweck können die erfindungsgemäßen Zusammensetzungen auch durch Wahl geeigneter Zusatzstoffe, eingesetzt werden zur kosmetischen und dermatologischen /therapeutischen Behandlung von Haut, Haar oder Schleimhaut. Die Zusammensetzungen können insofern als Creme, Lotion, Milch, Salbe, Gele, Sprays vorliegen und angewendet werden als solche zur Pflege, Reinigung (z. B. Dusche, Maske, Creme).

Besonders bevorzugt ist die Anwendung in Form einer Creme. Mittels der erfindungsgemäßen Kombination können die eingesetzten Transferproteine ihre Wirkung als Vehikel für lipophile / amphiphile Komponenten entfalten. Durch den Transfer von Lipiden erfolgt die Erneuerung der zellulären und extrazellulären Lipidstrukturen der Zellen, vor allem der Haut. Somit kann eine gute Transferrate zum Wirkstofftransport erzielt werden, ohne dass aufwendige Verfahren hierfür, wie z. B. bei Liposomen, erforderlich wären. So ist neben der Aufrechterhaltung von intakten, nicht starr vernetzten Lipidstrukturen, wie sie besonders im Alter auftreten, auch eine verbesserte Behandlung von Hautfunktionsstörungen möglich, da durch gezielten Transport einer jeweils benötigten Lipidkomponente eine rasche Behebung der Störung erfolgen kann. Umgekehrt können unerwünschte Lipidische Stoffe mittels dieses Systems entfernt werden. So kann z. B. neben der allgemeinen kosmetischen Anwendung für Reinigung, Pflege, Erscheinungen der Altershaut, auch ein therapeutischer Einsatz z. B. bei entzündlichen Zuständen, Reizzuständen der Haut z. B. infolge Allergie, oder Hautverbrennungen, bei Reizzuständen der Atemwege z. B. infolge Allergie , oder Irritationen vorgenommen werden. Insbesondere können mit den erfindungsgemäßen Zusammensetzungen bei topischer externer Anwendung Hautzellstrukturen von alternder, geschädigter, insbesondere Licht geschädigter, trockener, empfindlicher, entzündlicher Haut von Säugern, insbesondere Menschen, beeinflusst werden.

### Herstellung der Produkte

Die erfindungsgemäßen Zusammensetzungen werden hergestellt, indem man die wasserlöslichen bzw. wasserdispergierbaren Bestandteile in Wasser einarbeitet, analog die Lipidphase bereitet und beide Phasen bei geeigneten Temperaturen wie z. B. 20 bis 80° C, bevorzugt 30 bis 60° C bis Raumtemperatur so durchmischt, dass eine stabile Emulsion erhalten wird und ggf. bei Raumtemperatur temperaturlabile Phasen unter Rühren hinzufügt. Die Emulgierung erfolgt so, dass einerseits eine hohe Dispersität der kohärenten Phase erreicht wird und andererseits eine Depolymerisation der Schutzkolloide durch z. B. zu hohe Scherkräfte verhindert wird.

### Beispiele

Der Erfindung wird anhand der nachfolgenden Beispiele näher erläutert.

### Beispiel 1 O/W- Emulsion

| **Pos.** | **INCI** | **% [ W/W]** |
|---|---|---|
| **Fettphase** | | |
| 1 | Stearyl Alcohol | 2,0 |
| 2 | Glyceryl Stearat | 2,0 |
| 3 | Isopropyl Stearat | 6,0 |
| 4 | Persea Gratissima (Avocadoöl) | 3,0 |
| 5 | Dicaprylyl Ether | 3,0 |
| 6 | Squalane | 5,0 |
| 7 | Tocopheryl, Hydrogenanted Palm Glycerides Citrate | 0,05 |

| **Wasserphase** | | |
|---|---|---|
| 8 | Aqua | Ad 100 |
| 9 | Glycerin | 2,0 |
| 10 | Butylene Glykol | 2,0 |
| 11 | Disodium EDTA | 0,1 |
| 12 | Sodium Caseinate | 3,0 |
| 13 | Hydrolyzed Milk Protein | 0,5 |
| 14 | Avena Sativa (Haferprotein) | 0,5 |

| **Wirkstoffphase** | | |
|---|---|---|
| 15 | Cyclomethicone | 1,0 |
| 16 | Tocopheryl Acetate | 1,0 |
| 17 | Konservierungsstoffe | q.s. |
| 18 | Parfüm | q.s. |

## Patentansprüche

1. Extern applizierbare Zusammensetzung, enthaltend wenigstens ein Lipidtransferprotein, sowie wenigstens eine lipophilie und / oder amphiphile Komponente und Wasser, **dadurch gekennzeichnet, dass** die Zusammensetzung wenigstens ein emulgierendes Schutzkolloid aufweist, das ausgewählt ist aus natürlichen Hydrokolloiden, umfassend Caseinate, Proteinderivate tierischer Natur, Gelatine, Albumine; tierisch - marinen Kolloiden und Derivaten hiervon; oder synthetischen oder partialsynthetisch hergestellten Kolloiden oder Mischungen hiervon und dass das oder die Lipidtransferproteine ausgewählt ist /sind aus hydrolysiertem Milchprotein in Kombination mit Haferprotein bzw. Hydrolysat hiervon.

2. Zusammensetzung gemäß Anspruch 1, **dadurch gekennzeichnet, dass** Haferprotein bzw. Hydrolysat mit einer mittleren Molekülmasse von 4000 - 10000 D und hydrolysiertes Milchprotein mit einer mittleren Molekülmasse von 2000 bis 4000 D ausgewählt ist.

3. Zusammensetzung gemäß Anspruch 1 oder 2, **dadurch gekennzeichnet, dass** die lipophile und / oder amphiphile Komponente(n) ausgewählt ist (sind) aus Lecithinen, Cholesterinen, Phospholipiden, Sphingolipiden, Gangliosiden, Cerebrosiden, Ceramiden lipophilen oder amphiphilen Pflanzenstoffen, Glyceriden, Kohlenwasserstoffen, natürlichen oder synthetischen Fetten, Ölen, Wachsen, Fettalkoholen, Fettsäurestern, Fettsäurepartialestern, Silikonölen, Silikonwachsen oder Mischungen hiervon.

4. Zusammensetzung gemäß einem der Anspruche 1 bis 3, **dadurch gekennzeichnet, dass** die lipophile und / oder amphiphile Komponente(n) ausgewählt ist (sind) aus je einer oder mehreren Substanzen der Gruppe aus Kohlenwasserstoffen, natürlichen oder synthetischen Fetten, Ölen, Wachsen, Fettsäureester, Fettalkoholether, Polysiloxanverbindungen, Triglyceride, in Kombination mit je einer oder mehreren Substanzen aus der Gruppe aus Lecithinen, Sphingomyelinen, Cholesterinen, Phospholipiden, Sphingolipiden, Gangliosiden, Cerebrosiden, lipophilen oder amphiphilen Pfianzenstoffen, Mono-/Diglyceride von C₁₂₋₂₂- Fettsäuren, C₁₂₋₂₂Fettalkohole, C₁₂₋₂₂₋Fettsäurepartialester von C₂₋₆-Alkoholen, Polyol - C₁₂₋₂₂- Fettsäureester.

5. Zusammensetzung gemäß einem der Ansprüche 1 bis 4, **dadurch gekennzeichnet, dass** 0,01 Gew. % bis 40 Gew. % Zusatzstoffe enthalten sind.

6. Zusammensetzung gemäß einem der Ansprüche 1 bis 5, **dadurch gekennzeichnet, dass** 0,01 bis 10 Gew. % eines oder mehrerer Lipidtransferproteine, 0,01 bis 40 Gew. % einer oder mehrerer lipophiler und /oder amphiphiler Komponenten, 0,01 bis 10 Gew. % eines oder mehrerer Schutzkolloide, 0,01 bis 30 Gew. Zusatzstoffe und als Rest Wasser enthalten ist.

7. Zusammensetzung gemäß einem der Ansprüche 1 bis 6, **dadurch gekennzeichnet, dass** das Schutzkolloid ausgewählt ist aus Substanzen natürlichen, insbesondere tierischen Ursprungs.

8. Zusammensetzung gemäß einem der Ansprüche 1 bis 7, **dadurch gekennzeichnet, dass** das oder die Lipidtransferproteine in einer Menge von 0,01 bis 8 Gew. % vorliegen.

9. Zusammensetzung gemäß einem der Ansprüche 1 bis 8, **dadurch gekennzeichnet, dass** sie in Form einer Creme, Lotion, Milch, Salbe, Gels, Spray vorliegt.

10. Verwendung einer Zusammensetzung gemäß einem der Ansprüche 1 bis 9 zur Herstellung von Mitteln zur Beeinflussung von Lipidzelistrukturen.

11. Verwendung gemäß Anspruch 10 zur Herstellung von glitteln zur Reinigung, Pflege und/ oder der prophylaktischen oder therapeutischen Behandlung der Haut von Säugern.

12. Verwendung gemäß Anspruch 10 oder 11 zur Herstellung von Mitteln. zur Verbesserung der Hautzellstruktur von alternder, geschädigter, insbesondere Licht geschädigter, trockener, empfindlicher, entzündlicher Haut von Säugem.

13. Verwendung einer Zusammensetzung gemäß einem der Ansprüche 1 bis 9 zur kosmetischen Behandlung der Haut.

14. Verwendung gemäß Anspruch 13, **dadurch kennzeichnet, dass** die kosmetische Behandlung eine Reinigung, eine Pflege oder Sonnenschutz ist.

15. Verwendung einer Zusammensetzung gemäß Anspruch 14 als Duschpräparat, Pflegecreme oder Lotion.

16. Verwendung einer Zusammensetzung gemäß einem der Ansprüche 1 bis 9 zur Herstellung eines Mittels zur therapeutischen Behandlung der Haut von Säugem

17. Verwendung gemäß Anspruch 16 zur Verbesserung der Hautzellstruktur von alternder, geschädigter, insbesondere Licht geschädigter, trockener, empfindlicher, entzündlicher Haut von Säugem.

## Claims

1. Composition that can be applied externally, containing at least one lipid transfer protein as well as at least one lipophilic and/or amphiphilic component and water, **characterised in that** the composition has at least one emulsifying protective colloid selected from amongst natural hydrocolloids, including caseinates, protein derivatives of an animal nature, gelatins, albumins; animal-marine colloids and derivatives thereof; or synthetically or partially synthetically produced colloids or mixtures thereof, and **in that** the lipid transfer protein(s) is/are selected from amongst hydrolysed milk protein in conjunction with oat protein or a hydrolysate thereof.

2. Composition according to claim 1, **characterised in that** oat protein or hydrolysate having an average molecular mass of from 4,000 to 10,000 D is selected and hydrolysed milk protein, having an average molecular mass of from 2,000 to 4,000 D is selected.

3. Composition according to either claim 1 or claim 2, **characterised in that** the lipophilic and/or amphiphilic component(s) is/are selected from lecithins, cholesterols, phospholipids, sphingolipids, gangliosides, cerebrosides, ceramides, lipophilic or amphiphilic plant substances, glycerides, hydrocarbons, natural or synthetic fats, oils, waxes, fatty alcohols, fatty acid esters, fatty acid partial esters, silicone oils, silicone waxes, or mixtures thereof.

4. Composition according to any one of claims 1 to 3, **characterised in that** the lipophilic and/or amphiphilic component(s) is/are selected from one or more substances of the group of hydrocarbons, natural or synthetic fats, oils, waxes, fatty acid esters, fatty alcohol ethers, polysiloxane compounds, triglycerides, in each instance, in combination with one or more substances from the group of lecithins, sphingomyelins, cholesterols, phospholipids, sphingolipids, gangliosides, cerebrosides, lipophilic or amphiphilic plant substances, mono/diglycerides of C₁₂₋₂₂ fatty acids, C₁₂₋₂₂ fatty alcohols, C₁₂₋₂₂ fatty acid partial esters of C₂₋₆ alcohols, polyol-C₁₂₋₂₂ fatty acid esters.

5. Composition according to any one of claims 1 to 4, **characterised in that** it contains 0.01 % by weight to 40 % by weight of additives.

6. Composition according to any one of claims 1 to 5, **characterised in that** it contains 0.01 to 10 % by weight of one or more lipid transfer proteins, 0.01 to 40 % by weight of one or more lipophilic and/or amphiphilic components, 0.01 to 10 % by weight of one or more protective colloids, 0.01 to 30 % by weight of additives and the remainder is water.

7. Composition according to any one of claims 1 to 6, **characterised in that** the protective colloid is selected from amongst substances having a natural origin, in particular an animal origin.

8. Composition according to any one of claims 1 to 7, **characterised in that** the content of lipid transfer protein(s) is from 0.01 to 8 % by weight.

9. Composition according to any one of claims 1 to 8, **characterised in that** it is in the form of a cream, lotion, milk, ointment, gel, spray.

10. Use of a composition according to any one of claims 1 to 9 for the production of agents for influencing lipid cell structures.

11. Use according to claim 10 for the production of agents for cleansing, care, and/or the prophylactic or therapeutic treatment of the skin of mammals.

12. Use according to either claim 10 or claim 11 for the production of agents for improving the skin cell structure of aging, damaged, particularly light-damaged, dry, sensitive, inflamed skin of mammals.

13. Use of a composition according to any one of claims 1 to 9 for cosmetic treatment of the skin.

14. Use according to claim 13, **characterised in that** the cosmetic treatment is cleansing, care or sun protection.

15. Use of a composition according to claim 14 as a shower preparation, care cream or lotion.

16. Use of a composition according to any one of claims 1 to 9 for the production of an agent for therapeutically treating the skin of mammals.

17. Use according to claim 16 for improving the skin cell structure of ageing, damaged, particularly light-damaged, dry, sensitive, inflamed skin of mammals.

## Revendications

1. Composition à application externe, contenant au moins une protéine de transfert lipidique et au moins un composant lipophile et/ou amphiphile et de l'eau, **caractérisée en ce que** la composition présente au moins un colloïde de protection émulsifiant qui est choisi parmi des hydrocolloïdes naturels, comprenant des caséinates, des dérivés protéiques d'origine animale, la gélatine, l'albumine; des colloïdes animaux-marins et leurs dérivés ; ou bien des colloïdes préparés par voie synthétique ou par voie partiellement synthétique ou encore leurs mélanges, et **en ce que** la protéine ou les protéines de transfert lipidique est ou sont choisie(s) parmi des protéines du ait hydrolysée en combinaison avec de la protéine d'avoine, respectivement un hydrolysat de cette dernière.

2. Composition selon la revendication 1, **caractérisée en ce qu'**on choisit de la protéine d'avoine respectivement un de ses hydrolysats possédant une masse moléculaire moyenne de 4000 à 10.000 D et de la protéine du lait hydrolysée, possédant une masse moléculaire moyenne de 2000 à 4000 D.

3. Composition selon la revendication 1 ou 2, **caractérisée en ce que** le ou les composants lipophile(s) et/ou amphiphile(s) est/sont choisi(s) parmi les lécithines, les cholestérols, les phospholipides, les sphingolipides, les gangliosides, les cérébrosides, les céramides, des substances végétales lipophiles ou amphiphiles, des glycérides, des hydrocarbures, des graisses naturelles ou synthétiques, des huiles, des cires, des alcools gras, des esters d'acides gras, des esters partiels d'acide gras, des huiles de silicone, des cires de silicone, ou leurs mélanges.

4. Composition selon l'une quelconque des revendications 1 à 3, **caractérisée en ce que** le/les composants lipophile(s) et/ou amphiphile(s) est/sont choisi(s) à partir respectivement d'une ou de plusieurs substances faisant partie du groupe des hydrocarbures, des graisses naturelles ou synthétiques, des huiles, des cires, des esters d'acides gras, des éthers d'alcools gras, des composés polysiloxane, des triglycérides, en combinaison avec respectivement une ou plusieurs substances faisant partie du groupe des lécithines, des sphingomyélines, des cholestérols, des phospholipides, des sphingolipides, des gangliosides, des cérébrosides, des substances végétales lipophiles ou amphiphiles, des mono-/diglycérides d'acides gras en C₁₂-C₂₂, des alcools gras en C₁₂-C₂₂, des esters partiels d'acides gras en C₁₂-C₂₂ d'alcools en C₂-C₆, d'esters d'acides gras en C₁₂-C₂₂ de polyols.

5. Composition selon l'une quelconque des revendications 1 à 4, **caractérisée en ce qu'**elle contient des additifs à concurrence de 0,01 % en poids à 40 % en poids.

6. Composition selon l'une quelconque des revendications 1 à 5, **caractérisée en ce qu'**elle contient une ou plusieurs protéines de transfert lipidique à concurrence de 0,01 à 10 % en poids, un ou plusieurs composants lipophiles et/ou amphiphiles à concurrence de 0,01 à 40 % en poids, un ou plusieurs colloïdes de protection à concurrence de 0,01 à 10 % en poids, des additifs à concurrence de 0,01 à 30 % en poids, le reste étant de l'eau.

7. Composition selon l'une quelconque des revendications 1 à 6, **caractérisée en ce que** le colloïde de protection est choisi parmi des substances d'origine naturelle, en particulier d'origine animale.

8. Composition selon l'une quelconque des revendications 1 à 7, **caractérisée en ce que** la ou les protéines de transfert lipidique sont présentes en une quantité de 0,01 à 8 % en poids.

9. Composition selon l'une quelconque des revendications 1 à 8, **caractérisée en ce qu'**elle est présente sous la forme d'une crème, d'une lotion, d'un lait, d'un onguent, d'un gel, d'un spray.

10. Utilisation d'une composition selon l'une quelconque des revendications 1 à 9, pour la préparation d'agents destinés à influencer des structures de cellules lipidiques.

11. Utilisation selon la revendication 10, pour la préparation d'agents destinés à la purification, aux soins et/ou au traitement prophylactique ou thérapeutique de la peau de mammifères.

12. Utilisation selon la revendication 10 ou 11, pour la préparation d'agents destinés à améliorer la structure des cellules cutanées d'une peau vieillissante, abîmée, en particulier abîmée sous l'effet de la lumière, sèche, sensible, enflammée de mammifères.

13. Utilisation d'une composition selon l'une quelconque des revendications 1 à 9, pour le traitement cosmétique de la peau.

14. Utilisation selon la revendication 13, **caractérisée en ce que** le traitement cosmétique est une purification, un soin ou une protection solaire.

15. Utilisation d'une composition selon la revendication 14, comme préparation de douche, comme crème de soins ou comme lotion.

16. Utilisation d'une composition selon l'une quelconque des revendications 1 à 9, pour la préparation d'un agent destiné au traitement thérapeutique de la peau de mammifères.

17. Utilisation selon la revendication 16, pour améliorer la structure de cellules cutanées d'une peau vieillissante, abîmée, en particulier abîmée sous l'effet de la lumière, sèche, sensible, enflammée de mammifères.
